# EUROPÄISCHE PATENTSCHRIFT

(11) **EP 0 614 877 B1**
(45) Veröffentlichungstag und Bekanntmachung des Hinweises auf die Patenterteilung: **20.08.1997**
(21) Anmeldenummer: 94102978.7
(22) Anmeldetag: 28.02.1994
(51) Int. Cl.: C07C 68/06, C07C 69/96

(54) **Verfahren zur kontinuierlichen Herstellung von Arylcarbonaten**
Process for the continuous preparation of aryle carbonates
Procédé de préparation continue de carbonates d'aryle

(30) Priorität: 12.03.1993 DE 4307852; 17.05.1993 DE 4316428
(43) Veröffentlichungstag der Anmeldung: 14.09.1994
(73) Patentinhaber: BAYER AG, 51368 Leverkusen (DE)
(72) Erfinder: Rechner, Johann, Dr., D-47800 Krefeld (DE); Schön, Norbert, Dr., D-47800 Krefeld (DE); Wagner, Paul, Dr.r., D-40597 Düsseldorf (DE); Buysch, Hans-Josef, Dr., D-47809 Krefeld (DE); Kabelac, Stephan, Dr., D-40764 Langenfeld (DE)

(56) Entgegenhaltungen:
- EP-A- 0 461 274
- WO-A-92/18458
- DE-A- 3 308 921
- PATENT ABSTRACTS OF JAPAN vol. 016, no. 581 (C-1012)21. Dezember 1992 & JP-A-04 230 242 (ASAHI CHEM IND CO LTD) 19. August 1992
- PATENT ABSTRACTS OF JAPAN vol. 016, no. 584 (C-1013)24. Dezember 1992 & JP-A-04 235 951 (ASAHI CHEM IND CO LTD) 25. August 1992
- PATENT ABSTRACTS OF JAPAN, vol. 016, no. 581 (C-1012), 21. Dezember 1992 & JP-A-04 230 242
- PATENT ABSTRACTS OF JAPAN, vol. 016, no. 584 (C-1013), 24. Dezember 1992 & JP-A-04 235 951

## Beschreibung

Die Erfindung betrifft ein kontinuierliches Verfahren zur Herstellung von Arylcarbonaten aus mindestens eine aliphatische Estergruppe enthaltenden Carbonaten und Phenolen einerseits und aus Alkylarylcarbonaten andererseits durch katalysierte Umesterung, wobei die Umsetzung in einer oder mehreren Blasensäulen durchgeführt wird.

Die Herstellung von aromatischen und aliphatisch-aromatischen Kohlensäureestern (Carbonaten) durch Umesterung, ausgehend von aliphatischen Kohlensäureestern und Phenolen, ist im Prinzip bekannt. Dabei handelt es sich um eine Gleichgewichtsreaktion, wobei die Lage des Gleichgewichts fast vollständig in Richtung der aliphatisch substituierten Carbonate verschoben ist. Daher ist es verhältnismäßig leicht, aus aromatischen Carbonaten und Alkoholen aliphatische Carbonate herzustellen. Um die Reaktion im umgekehrten Sinne in Richtung aromatischer Carbonate durchzuführen, ist es jedoch notwendig, das sehr ungünstig liegende Gleichgewicht effektiv zu verschieben, wobei nicht nur sehr aktive Katalysatoren, sondern auch eine günstige Verfahrensweise zur Anwendung gelangen müssen.

Zur Umesterung von aliphatischen Kohlensäureestern mit Phenolen sind eine Vielzahl von effektiven Katalysatoren, wie beispielsweie Alkalihydroxide, Lewissäure-Katalysatoren aus der Gruppe der Metallhalogenide (DE-OS 2 528 412 und 2 552 907), Organozinnverbindungen (EP 0 000 879, EP 0 000 880, DE-OS 3 445 552, EP 0 338 760), Bleiverbindungen (JP 57/176 932), Lewis-Säure/-Protonensäure-Katalysatoren (DE-OS 3 445 553) empfohlen worden.

In den bekannten Verfahren wird die Umesterung in einem chargenweise betriebenen Reaktor drucklos oder unter Druck, gegebenenfalls mit einer zusätzlichen Trennkolonne, durchgeführt. Dabei werden auch mit den aktivsten Katalysatoren Reaktionszeiten von vielen Stunden bis zum Erreichen auch nur mittlerer Umsätze von ungefähr 50 % an Phenol benötigt. So werden bei der chargenweise betriebenen Umesterung von Phenol mit Diethylcarbonat bei 180°C unter Verwendung verschiedener Organozinnverbindungen, wie sie in DE-OS 3 445 552 beschrieben werden, Ausbeuten an Diphenylcarbonat in einer Größenordnung von mehr als 20 % erst nach ca. 24-stündiger Reaktionszeit erreicht; bei der chargenweise betriebenen Umesterung von Phenol und Dimethylcarbonat mit Hilfe von Organozinnkatalysatoren, wie in EP 0 000 879 beschrieben, beträgt der Phenolumsatz nach 30 h 34 % des theoretischen Wertes.

Das bedeutet, daß aufgrund der ungünstigen thermodynamischen Voraussetzungen die beschriebenen, chargenweise betriebenen Umesterungsreaktionen selbst bei Verwendung sehr aktiver Katalysatorsysteme im Sinne eines technischen Prozesses nur sehr unvorteilhaft durchführbar sind, da sehr schlechte Raum-Zeit-Ausbeuten und hohe Verweilzeiten bei hohen Reaktionstemperaturen erforderlich sind.

Solche Verfahrensweisen sind auch deshalb besonders unvorteilhaft, da selbst mit sehr selektiven Umesterungskatalysatoren bei den hohen Temperaturen und langen Verweilzeiten von vielen Stunden ein merklicher Anteil an Nebenreaktionen auftritt, beispielsweise die Etherbildung unter Abspaltung von Kohlendioxid.

Es wurde daher versucht, das Reaktionsgleichgewicht durch Adsorption des bei der Umesterung entstehenden Alkohols an Molekularsieben möglichst schnell in Richtung der Produkte zu verschieben (DE-OS 3 308 921). Aus der Beschreibung dieser Verfahrensweise zeigt sich, daß zur Adsorption des Reaktionsalkohols eine große Menge an Molekularsieb benötigt wird, die die Menge an freiwerdendem Alkohol um mindestens das fünffache überschreitet. Weiterhin müssen die eingesetzten Molekularsiebe schon nach kurzer Zeit regeneriert werden, und die Umwandlungsrate zu den Alkylarylcarbonat-Zwischenprodukten ist relativ gering. Auch dieses Verfahren erscheint deshalb als technisch und wirtschaftlich nicht vorteilhaft anwendbar.

Ein kontinuierlicher Umesterungsprozeß zur Herstellung von aromatischen Carbonaten, bei dem die Reaktion in einer oder in mehreren hintereinander geschalteten mehrstufigen Destillationskolonnen durchgeführt wird, ist in EP-A 0 461 274 beschrieben. Dabei werden zunächst Phenole mit Dialkylcarbonaten zu Arylcarbonatgemischen umgesetzt, die im wesentlichen Alkylarylcarbonate enthalten. In einer zweiten, bevorzugt nachgeschalteten mehrstufigen Destillationskolonne werden diese dann zu den gewünschten Diarylcarbonat-Endprodukten weiter umgesetzt. Die Anmelderin betont die Effektivität und die Selektivität ihrer Verfahrensweise.

Als Kriterium für die Beurteilung eines Prozesses dient dem Fachmann neben Umsatz und Selektivität die Angabe der Raum-Zeit-Ausbeute (RZA), da sie die Ausbeute an Produkt pro benutztem Apparatevolumen beschreibt. Am Beispiel der Umesterung von Dimethylcarbonat (DMC) mit Phenol zu Methylphenylcarbonat (MPC) und Diphenylcarbonat (DPC) zeigt die Anmelderin von EP 0 461 274 einen Vergleich der Batchfahrweise in einem Autoklaven (Vergleichsbeispiel 1) mit einer Fahrweise in einer mehrstufigen Destillationskolonne (Beispiel 1). Hierbei wird lediglich eine Steigerung der RZA von 5 auf 8 g der Summe von DPC + MPC/l.h erzielt, wie sich aus den Beispielen leicht errechnen läßt. Die RZA sind in beiden Beispielen vergleichsweise gering, lediglich die MPC-Selektivität ist bei der Fahrweise in einer mehrstufigen Destillationskolonne von 94 % auf 97 % angestiegen.

Diese Ergebnisse werden bereits unter optimalen Bedingungen mit den besten Umesterungskatalysatoren bei hohen Temperaturen und erhöhtem Druck erzielt, so daß weitere Verbesserungen nicht möglich zu sein scheinen.

Die Weiterreaktion der Alkylarylcarbonate zu Diarylcarbonaten verläuft in der angegebenen Verfahrensweise, wie sie aus den Beispielen hervorgeht, im Sinne einer Disproportionierungsreaktion. So ist es nicht verwunderlich, daß bei dieser, im Vergleich zur ersten Umesterungsstufe leichter ablaufenden Reaktion, wesentlich bessere RZA erzielt werden.

Für die zweite Umesterungsstufe vergleicht EP 0 461 274 in einer Gegenüberstellung die Umesterung von Methylphenylcarbonat (MPC) zum Diphenylcarbonat (DPC) in der Batchfahrweise im Autoklaven (Vergleichsbeispiel 2) mit der Durchführung in einer mehrstufigen Destillationskolonne (Beispiel 11). Hierbei zeigen die aus den dort gemachten Angaben berechneten RZA für DPC sogar eine Verringerung der Effektivität von 144 g DPC/l.h auf 133 g DPC/l.h. Lediglich die Bildung des Nebenprodukts Anisol tritt in geringerem Maße auf.

Aufgrund dieser Angaben und dem erheblich höheren apparativen Aufwand, muß die hier aufgezeigte Verbesserung äußerst skeptisch beurteilt werden.

Weiterentwicklungen des Verfahrens gemäß EP 0 461 274 finden sich in JP 04/230 242 (1992) und in JP 04/235 951 (1992), in denen die mehrstufige Destillationskolonne zusätzlich mit einfachen, außerhalb der Kolonne befindlichen Durchfluß-/Verweilzeit-Reaktoren ausgerüstet ist.

In WO 92/18458 wird die Herstellung von Diarylcarbonaten durch Umesterung in Reaktoren beschrieben, in denen durch Einbau eines Wärmetauschers eine Konvektion zur Durchmischung der Reaktionspartner erzeugt wird. Aromatische Hydroxyverbindung wird in diesem Verfahren in mehrere Reaktoren eingespeist.

Ziel einer Verbesserung der erfindungsgemäßen Umesterungsreaktion sollte deshalb vor allem eine Steigerung der RZA, vor allem der Umesterungsstufen mit Phenol sein, wobei die Selektivität des gesamten Prozesses nicht herabgesetzt werden sollte.

Überraschenderweise wurde nun gefunden, daß die Steigerung der RZA in einem kontinuierlich geführten Umesterungsverfahren bei sehr hoher Selektivität in Blasensäulen gelingt. Dies war besonders überraschend, da Blasensäulen für diese Reaktion vermeintlich ungeeignete Reaktoren sind, in ihren Eigenschaften den absatzweise betriebenen Reaktoren ähneln und in ihnen daher gegenüber einer Destillationskolonne längere Flüssigkeitsverweilzeiten vorliegen, die die Gefahr der Bildung von Nebenprodukten erhöhen. Hohe RZA bei der erfindungsgemäßen Carbonat-Umesterung gelingen in Blasensäulenreaktoren bereits bei niedrigen Temperaturen und auch bei druckloser Fahrweise. Die für diese Reaktion ungewöhnlichen Reaktoren sind dem Fachmann ansonsten vor allem für Absorptionsprozesse, beispielsweise in der Abgasreinigung, bekannt.

Blasensäulenreaktoren sind einfache Apparate ohne Rührorgane, bei denen sich Temperatur, Druck und insbesondere die Flüssigkeitsverweilzeiten in weiten Bereichen einstellen lassen, so daß eine variable Verfahrensweise zur Verfügung steht.

Die Erfindung betrifft demnach ein Verfahren zur Herstellung eines aromatischen Carbonats der Formel

R¹-O-CO-O-R² (I)

in der
- R²: Phenyl oder Naphthyl sowie ein- bis dreifach durch geradkettiges oder verzweigtes C₁-C₄-Alkyl, geradkettiges oder verzweigtes C₁-C₄-Alkoxy, Cyano und/oder Halogen substituiertes Phenyl bzw. Naphthyl bedeutet, und
- R¹: unabhängig von R² den Bedeutungsumfang von R² annimmt oder geradkettiges oder verzweigtes C₁-C₆-Alkyl bedeutet,
durch katalysierte Umsetzung von 0,1 bis 10 mol, bevorzugt 0,2 bis 5 mol, besonders bevorzugt 0,5 bis 3 mol eines organischen Carbonats mit mindestens einer aliphatischen Estergruppe der Formel

R¹-O-CO-O-R³ (II)

in der
- R³: geradkettiges oder verzweigtes C₁-C₆-Alkyl bedeutet und
- R¹: den obigen Bedeutungsumfang hat,
mit 1 mol einer phenolischen Verbindung der Formel

R²-OX (III)

in der
- R²: den obigen Bedeutungsumfang hat und
- X: für Wasserstoff oder für -CO-O-C₁-C₆-Alkyl mit geradkettiger oder verzweigter Alkylgruppe steht,
in Gegenwart eines an sich bekannten Umesterungskatalysators bei 80 bis 350°C, das dadurch gekennzeichnet ist, daß die Umsetzung in einem Blasensäulenreaktor oder einer Kaskade aus mindestens zwei Blasensäulen so durchgeführt wird, daß die phenolische Verbindung der Formel (III) in flüssiger Form in die erste Blasensäule und das organische Carbonat der Formel (II) flüssig oder gasförmig gleichzeitig in jede einzelne, bevorzugt aber nur in die letzte Blasensäule, eindosiert werden, wobei bei flüssiger Eindosierung eine Verdampfung von (II) in der Blasensäule erfolgt, und aus der letzten Blasensäule die Reaktionsprodukte der Formel (I) in flüssiger Form und gleichzeitig am oberen Ende jeder einzelnen Blasensäule, bevorzugt am oberen Ende der ersten Blasensäule, gasförmig die Produkte der Formel

R³-OX (IV)

in der R³ und X die genannte Bedeutung haben,
entnommen werden.

Die Umesterung nach dem erfindungsgemäßen Verfahren umfaßt mehrere Reaktionen, wie die folgenden Gleichungen in verallgemeinerter Form zeigen (Alk=Alkyl; Ar=Aryl):

Alk-O-CO-O-Alk+Ar-OH→Alk-O-CO-O-Ar+Alk-OH (Gleichung 1)

Alk-O-CO-O-Ar + Ar-OH→Ar-O-CO-O-Ar + Alk-OH (Gleichung 2)

2 Ar-OCO-O-Alk→Ar-OCO-O-Ar+Alk-OCO-O-Alk (Gleichung 3)

Bei der Bildung eines Diarylcarbonats erfolgt die Umesterung von den aliphatischen zu den aromatischen Estergruppen in zwei Stufen, wobei ein Alkylarylcarbonat im Sinne der Gleichung 1 als Produkt der ersten Umesterungsstufe durchlaufen wird.

Die Gleichung 3 zeigt ferner eine Disproportionierungsreaktion, in welcher aus einem gemischten Alkylarylcarbonat sowohl das symmetrische Dialkylcarbonat als auch das gewünschte symmetrische Diarylcarbonat entstehen. Es ist ferner möglich, das Alkylarylcarbonat als das gewünschte Reaktionsprodukt zu erhalten, also im wesentlichen nur die erste Umesterungsstufe zu betreiben. Noch weiterhin ist möglich, durch Einsatz von Gemischen verschiedener Phenole auch unsymmetrische Diarylcarbonate zu erhalten.

Zum Einsatz gelangen Dialkylcarbonate mit gleichen oder verschiedenen aliphatischen Estergruppen mit geradkettigem oder verzweigtem C₁-C₆-Alkyl. Solche Dialkylcarbonate sind dem Fachmann bekannt und können nach bekannten Verfahren hergestellt werden. Aus ökonomischen Gründen wird man im allgemeinen von symmetrischen Dialkylcarbonaten ausgehen.

Geradkettiges oder verzweigtes C₁-C₆-Alkyl ist beispielsweise Methyl, Ethyl, Propyl, Isopropyl, Butyl, Isobutyl, Pentyl oder Hexyl, bevorzugt Methyl oder Ethyl, besonders bevorzugt Methyl.

Geradkettiges oder verzweigtes C₁-C₄-Alkoxy ist beispielsweise Methoxy, Ethoxy, Propoxy, Isopropoxy, Butoxy oder Isobutoxy, bevorzugt Methoxy.

Halogen ist beispielsweise Fluor, Chlor oder Brom, bevorzugt Fluor oder Chlor, besonders bevorzugt Chlor.

Die aromatische Estergruppe kann von einem Phenol oder einem Naphthol, bevorzugt von einem Phenol abgeleitet sein und in der angegebenen Weise ein- bis dreifach, bevorzugt ein- oder zweifach, besonders bevorzugt einfach substituiert sein. Der Cyano-Substituent tritt in der Regel nur einfach als Substituent auf, Ganz besondere Bedeutung hat das erfindungsgemäße Verfahren für die Umesterung von nicht substituiertem Phenol.

Erfindungsgemäß einsetzbare Phenole, die unter die Formel (III) fallen, wenn X für Wasserstoff steht, sind beispielsweise nicht substituiertes Phenol, o-, m- oder p-Kresol, o-, m- oder p-Chlorphenol, o-, m- oder p-Ethylphenol, o-, m- oder p-Propylphenol, o-, m- oder p-Methoxyphenol, 2,6-Dimethylphenol, 2,4-Dimethylphenol, 3,4-Dimethylphenol, 1-Naphthol und 2-Naphthol.

Bevorzugt einsetzbare phenolische Verbindungen sind demnach allgemein solche der Formel

R¹²-OH (V),

in der
- R¹²: Phenyl oder einfach durch C₁-C₄-Alkyl, C₁-C₄-Alkoxy oder Chlor substituiertes Phenyl bedeutet.

Hierunter ist das nicht substituierte Phenol besonders bevorzugt.

Als organische Carbonate mit mindestens einer aliphatischen Estergruppe werden bevorzugt symmetrische Dialkylcarbonate der Formel

R³-O-CO-O-R³ (VI),

in der
- R³: die angegebene Bedeutung hat,
eingesetzt.

Erfindungsgemäß einsetzbare Dialkylcarbonate sind beispielsweise Dimethylcarbonat, Diethylcarbonat, Dipropylcarbonat, Dibutylcarbonat und Dihexylcarbonat. Bevorzugt einsetzbare Dialkylcarbonate sind Dimethyl-und Diethylcarbonat, besonders bevorzugt Dimethylcarbonat (DMC).

Das organische Carbonat (II) mit mindestens einer aliphatischen Estergruppe kann als solches im erfindungsgemäßen Verfahren eingesetzt werden. Es ist jedoch möglich und stellt eine bevorzugte Variante dar, dieses organische Carbonat im Gemisch mit geringen Mengen des zugrundeliegenden Alkohols R³-OH einzusetzen. Der Alkohol R³-OH tritt im erfindungsgemäßen Verfahren als Spaltprodukt auf und stellt den Spezialfall der Formel (IV) mit X = H dar. Die Spaltprodukte Carbonat (X = -CO-O-C₂-C₆-Alkyl) und Alkohol (X = H) brauchen demnach zu einer Rückführung des Carbonats in das erfindungsgemäße Verfahren nicht vollständig getrennt zu werden; dies stellt einen energetischen Vorteil dar. Die Menge des im Gemisch mit dem Carbonat zulässigen Alkohols beträgt 0-5 Gew.-%, bevorzugt 0,1-3 Gew.-%, besonders bevorzugt 0,15-2 Gew.-%, bezogen auf die Menge des eingesetzten Carbonats. Die Untergrenze Null bezeichnet die Fahrweise mit reinem Carbonat.

Erfindungsgemäß herstellbare Diarylcarbonate sind beispielsweise Diphenylcarbonat, die symmetrisch und unsymmetrisch substituierten isomeren Biskresylcarbonate, die symmetrisch und unsymmetrisch substituierten isomeren Bis(chlorphenyl)-carbonate, die symmetrisch und unsymmetrisch substituierten isomeren Bis(methoxyphenyl)-carbonate, die symmetrisch und unsymmetrisch substituierten isomeren Bis(ethoxyphenyl)-carbonate, Bis(2,6-dimethylphenyl)-carbonat, Bis(2,4-dimethylphenyl)-carbonat, Di-1-naphthyl-carbonat und Di-2-naphthyl-carbonat, außerdem weitere unsymmetrisch substituierte Diarylcarbonate, beispielsweise die isomeren Kresyl-phenyl-carbonate, die isomeren (Chlorphenyl)phenyl-carbonate, die isomeren (Methoxyphenyl)phenyl-carbonate, die isomeren Naphthyl-phenylcarbonate und 1-Naphthyl-2-naphthyl-carbonat.

Bevorzugte erfindungsgemäß herstellbare Diarylcarbonate sind solche der Formeln

R¹⁵-OCOO-R¹² (VII)

bzw.

R¹²-OCOO-R¹² (VIII),

in denen
- R¹² und R¹⁵: unabhängig voneinander den für R¹² weiter oben angegebenen Bedeutungsumfang haben.

Besonders bevorzugt herstellbares Diarylcarbonat ist Diphenylcarbonat.

Erfindungsgemäß herstellbare Alkylarylcarbonate sind beispielsweise C₁-C₆-Alkyl-phenyl-carbonate, wie Methylphenyl-carbonat, Ethyl-phenyl-carbonat, Propyl-phenylcarbonat, Butyl-phenyl-carbonat und Hexyl-phenyl-carbonat, C₁-C₆-Alkyl(o-, m-, p-kresyl)-carbonate, wie Methyl-(o-kresyl)-carbonat, Methyl-(p-kresyl)-carbonat, Ethyl-(o-kresyl)-carbonat, Ethyl-(p-kresyl)-carbonat, C₁-C₆-Alkyl-(o-, m-, p-Chlorphenyl)-carbonate, wie Methyl- oder Ethyl-(p-chlorphenyl)-carbonat und analoge Verbindungen. Besonders bevorzugt herstellbare Alkylarylcarbonate sind Methyl-phenyl-carbonat und Ethylphenyl-carbonat, ganz besonders bevorzugt Methyl-phenylcarbonat.

Als Blasensäulenreaktoren können im erfindungsgemäßen Verfahren folgende Typen eingesetzt werden: einfache Blasensäulen, Kaskaden aus einfachen Blasensäulen, Blasensäulen mit Einbauten und Kaskaden dieser Blasensäulen, wie z.B.: Blasensäulen mit parallelen Kammern, Kaskaden-Blasensäulen, Blasensäulen mit Packungen, Blasensäulen mit statischen Mischern, pulsierende Siebbodenblasensäulen, sowie weitere dem Fachmann bekannte Blasensäulenreaktoren (H. Gerstenberg, Chem. Ing. Tech. 61 (1979) Nr. 3, S. 208-216; W.-D. Deckwer, Reaktionstechnik in Blasensäulen, Otto Salle Verlag (1985)).

In der bevorzugten Ausführungsform kommen folgende Blasensäulenreaktoren oder Kaskaden von Blasensäulenreaktoren zum Einsatz: einfache Blasensäulen, Kaskaden-Blasensäulen, Blasensäulen mit parallelen Kammern und Blasensäulen mit statischen Mischern oder Packungen.

In einer weiteren bevorzugten Ausführungsform können auch Kombinationen sowohl der einzelnen Blasensäulenreaktoren in einer Kaskade von Blasensäulen als auch in einer Kaskaden-Blasensäule zum Einsatz kommen.

Zur Aufrechterhaltung einer möglichst homogenen Blasenströmung durch die Flüssigkeit können Verteilungs- und Redispergierorgane in den Blasensäulenreaktor entlang der Längsachse angebracht sein.

Als feste Redispergierorgane kommen Einlochböden, Lochplatten, Sieblochböden, sowie weitere dem Fachmann bekannte Einbauten zum Einsatz, die bei wirksamer Vermeidung von Rückvermischungen den Gegenstrom von Gasphase und Flüssigphase ermöglichen.

In den einzelnen Kaskaden-Blasensäulenreaktoren können nach der ersten Dispergierung der Gasphase weitere 0 bis 20, bevorzugt 1 bis 15 Redispergierorgane vorhanden seine Hierbei stellt eine Blasensäule mit 0 Redispergierorganen den Spezialfall einer einfachen Blasensäule dar. Die Gesamtzahl der Redispergierorgane in einer Kaskade von Blasensäulen kann somit 100, bevorzugt 75, besonders bevorzugt bis zu 60 betragen.

Bei der Gegenstromführung der Flüssig- und Gasphase in Kaskaden-Blasensäulen kann die Flüssigkeit entweder durch die Dispergierorgane strömen oder durch innere bzw. äußere Überlaufleitungen den darunter liegenden Blasensäulenabschnitten zuströmen.

Für die Erstdispergierung des gasförmigen Carbonats der Formel (II) in der flüssigen Phase bei der Eindosierung sind übliche Vorrichtungen wie poröse Sinterplatten, Lochplatten, Sieblochböden, Einsteckrohre, Düsen, Begasungsringe und weitere dem Fachmann bekannte Dispergiervorrichtungen einsetzbar.

Innerhalb einer Blasensäule oder im Falle der Verwendung einer Kaskade von Blasensäulen können auch innerhalb einer einzelnen Blasensäule verschiedene der oben genannten Dispergierorgane gleichzeitig, d.h. z.B. feste Einbauten neben Packungen vorliegen.

Der Flüssigkeits-Hold up in den Blasensäulenreaktoren beträgt mehr als 40 %, bevorzugt mehr als 50 % und besonders bevorzugt mehr als 75 % des verfügbaren Volumens.

Die Gasgeschwindigkeit, bezogen auf den leeren Reaktorquerschnitt, beträgt 0,1 bis 100 cm/s, bevorzugt 1 bis 50 cm/s und besonders bevorzugt 2 bis 30 cm/s.

Der Schlankheitsgrad der Blasensäulenreaktoren (Verhältnis von Länge zu Durchmesser) beträgt 1 bis 30, bevorzugt 1-20.

Für den Fall, daß Blasensäulenreaktoren mit parallelen Kammern verwendet werden, kann das Verhältnis von Länge zu Gesamtdurchmesser der Blasensäule von diesen Zahlenangaben abweichen, da hier die einzelnen Kammern zu berücksichtigen sind.

Zur Wärmezufuhr in die Blasensäulen sind außenliegende Heizungen, wie z.B. Mantelheizungen, Wärmeaustauscher für zwischenentnommene Flüssigkeiten oder innenliegende Wärmeaustauscher, wie z.B. parallele Einzelrohre, Querrohrbündel, Längsrohrbündel, Rohrspiralen, Rohrwendeln, Leitrohre mit Mantel und weitere dem Fachmann als Stand der Technik bekannte Wärmeaustauschvorrichtungen geeignet. In einer bevorzugten Ausführungsform können die innenliegenden Wärmeaustauscher zusätzlich Leitfunktionen für die Flüssigkeitsströmung und die Gasdispergierung übernehmen.

Zur Abtrennung der leichter flüchtigen Komponenten aus der am unteren Ende anfallenden flüssigen Phase kann eine Abtriebskolonne nach dem Stand der Technik installiert werden. Ebenso kann zur Reinigung der aus Dialkylcarbonat und dem betreffenden Alkohol bestehenden Gasphase von den aromatischen Hydroxyverbindung und den Umesterungsprodukten Alkylarylcarbonat und Diarylcarbonat das obere Ende der Blasensäule mit einer Verstärkungskolonne ausgerüstet sein.

In einer weiteren Verfahrensweise kann zusätzlich zu den Edukten ein unter den Reaktionsbedingungen inertes Lösungsmittel, das in der Blasensäule verdampft, oder Gas an beliebiger Stelle der Apparatur eingespeist werden. Solche inerte Lösungsmittel sind beispielsweise Kohlenwasserstoffe, wie Hexan, Heptan, i-Octan, Methyl-cyclopentan, Cyclohexan, Methylcyclohexan, Toluol, Xylole, Chlorbenzole, Tetralin, Dekalin etc. Als inerte Gase kommen beispielsweise Kohlendioxid, Stickstoff, Edelgase etc. in Frage. Diese inerten Lösungsmittel und Gase können auch zusammen mit dem gasförmigen oder in der Blasensäule zu verdampfenden Carbonat zudosiert und in einem weiten Konzentrationsbereich variiert werden.

In einigen Ausführungsformen kann es sinnvoll sein, auch das reine inerte Gas oder Lösungsmittel in eine oder mehrere Blasensäulen zu dosieren.

Für den Fall, wenn als aliphatisches Carbonat DMC verwendet wird, kann es vorteilhaft sein, ein inertes Lösungsmittel zu verwenden, welches mit Methanol ein Azeotrop bildet und dieses bevorzugt aus der Blasensäule entfernt Durch die Entfernung des Methanols aus dem Gleichgewicht wird der Fortgang des erfindungsgemäßen Verfahrens gefördert.

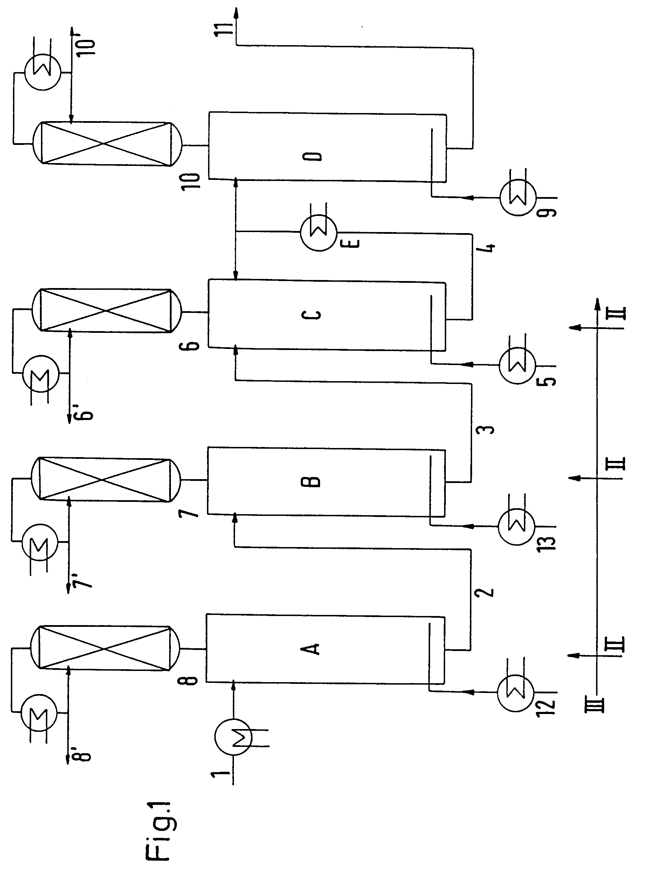

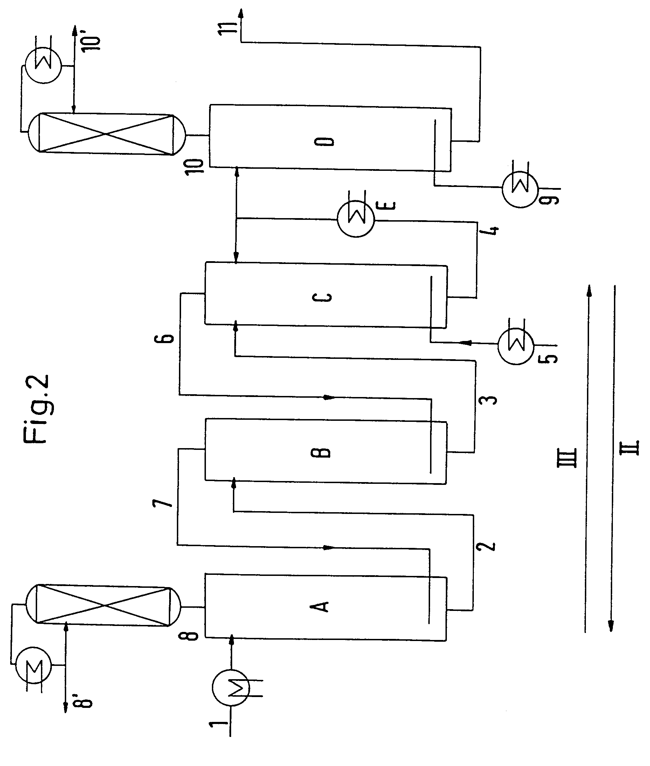

In Fig. 1 und 2 sind beispielhaft verschiedene Ausführungsformen der Erfindung gezeigt. Im Text angegebene Nummern und Buchstaben beziehen sich auf diese Fig.

Darin wird das erfindungsgemäße Verfahren bevorzugt unter Einsatz von 1 bis 18, besonders bevorzugt 2 bis 12 Blasensäulenreaktoren durchgeführt, wobei die Untergrenze 1 die Durchführung in einer einzelnen Blasensäule darstellt.

In der bevorzugten Ausführungsform kommt eine Kaskade von Kaskaden-Blasensäulenreaktoren zum Einsatz (Kaskaden-Blasensäulen). In Fig. 1 und 2 sind beispielhaft Arbeitsweisen mit 3 Blasensäulenreaktoren (A, B und C) dargestellt, wobei die erfindungsgemäße Arbeitsweise nicht auf diese Beispiele eingeschränkt werden soll. D und E stellen später erläuterte Verweilzeitbehälter für die Vervollständigung der Reaktion bzw. Abtriebsteile von Kolonnen für Stofftrennungen dar.

Die in die erste Blasensäule (A) eindosierte Reaktionskomponente der Formel (III) kann gegebenenfalls in einem vorgeschalteten Erhitzerelement auf die vorgesehene Reaktionstemperatur vorgeheizt werdend. Sie wird in flüssiger Form über Leitung (1) bevorzugt am oberen Ende der Blasensäule eingebracht.

Die aus der jeweiligen Blasensäule zu entnehmende Flüssigphase wird am unteren Ende entnommen und über die Leitungen (2), (3) oder (4) in die jeweils folgende Blasensäule B oder C am oberen Ende wieder eindosiert. Die Regelung des gewünschten Füllstandes in den kontinuierlich betriebenen Blasensäulenreaktoren erfolgt nach dem Stand der Technik.

Bei der Benutzung einer Blasensäulenkaskade kann die Gasphase (II) durch den kontinuierlich laufenden Flüssigkeitsstrom (III) + (I) entweder im Querstrom (Fig. 1) oder bevorzugt im Gegenstrom (Fig. 2) geschickt werden.

Querstrom bedeutet hierbei, daß die Edukte der Formel (II) jeweils an jedem Blasensäulenreaktor über die Leitungen (12), (13), (5) (Fig. 1) eindosiert werden und jeweils am oberen Ende jeder Blasensäule über die Leitungen (8), (7) und (6) (Fig. 1) wieder entnommen werden, d.h. die Edukte der Formel (II) durchströmen die Blasensäulenreaktoren quer zur Flußrichtung der Flüssigphase (III) + (I). Die Gesamtmenge der eindosierten Edukte der Formel (II) kann dabei beliebig auf die einzelnen Blasensäulenreaktoren aufgeteilt werden. In dem jeweiligen Blasensäulenreaktor wird hierbei bevorzugt die Gegenstromfahrweise von Flüssigphase und Gasphase realisiert.

Die bevorzugt zu verwendende Gegenstromfahrweise (Fig. 2) bedeutet, daß man die Edukte der Formel (II) in den letzten Blasensäulenreaktor (in Fig. 2 Reaktor C) eindosiert, kontinuierlich gegen die vom ersten Blasensäulenreaktor zum letzten Reaktor (C in Fig. 2) laufenden Flüssigphase führt und am oberen Ende des ersten Blasensäulenreaktors (A in Fig. 2) überschüssiges Edukt (II) sowie gebildetes Produkt (IV) entnimmt. Sofern (II) und (IV) ein Azeotrop bilden wie im Falle DMC/Methanol, kann es günstig sein, ein solches Azeotrop teilweise auch am oberen Ende von zwischenliegenden Reaktoren abzunehmen.

Die Edukte der Formel (II) und die gegebenenfalls zugegebene inerte Verbindung können in beiden Fällen entweder flüssig eindosiert und durch die vorhandene Flüssigphase verdampft werden oder bevorzugt in einem vorgeschalteten Apparat verdampft und gasförmig in die jeweilige Blasensäule eingebracht werden.

Es ist weiterhin auch möglich, die Edukte der Formel (II) zu einem Teil im Querstrom und zu einem anderen Teil im Gegenstrom zur Flüssigphase (III) + (I) strömen zu lassen.

Die am oberen Ende der jeweiligen Blasensäule zu entnehmenden Reaktionsprodukte der Formel (IV) können z.B. direkt gasförmig über (6'), (7') und (8') abgenommen werden.

Dabei ist es gegebenenfalls von Vorteil, durch geeignete Dephlegmierung oder/und durch eine aufgesetzte Kolonne vorher höhersiedende Reaktionsbestandteile z.B. Produkte der Formel (I) oder Edukte der Formel (III) abzutrennen und in die jeweilige Blasensäule zurückzuführen. Die Produkte der Formel (IV) können beispielsweise hierzu ohne Kondensation in eine geeignete Trennvorrichtung eingebracht werden Dies könnte im Falle der Umsetzung von Dimethylcarbonat mit Phenol eine Druckdestillationskolonne zur Trennung des anfallenden Dimethylcarbonat-Methanol-Gemisches sein, um möglichst wenig DMC im Kopfprodukt der Trennkolonne zu erhalten. Das dabei anfallende Dimethylcarbonat, das gegebenenfalls noch geringe Mengen Methanol enthält, kann als Edukt der Formel (II) in den Umesterungsprozess zurückgeführt werden.

Ebenso ist es möglich, die Produkte der Formel (IV), gegebenenfalls nach Abtrennung von höhersiedenden Reaktionsbestandteilen, wie oben beschrieben, abzunehmen und zu kondensieren. Eine Reinigung und Auftrennung des Produktstroms kann dann in geeigneter, dem Fachmann bekannter Art und Weise durchgeführt werden.

Der am letzten Reaktor, z.B. C in Figur 1 und 2, flüssig zu entnehmende Produktstrom kann gegebenenfalls in einem nachgeschalteten Abtriebsteil (E in Fig. 1 und 2) von leichtsiedenden Bestandteilen, z.B. den Edukten der Formel (II) oder den Produkten der Formel (IV), abgetrennt werden, die dann in die Reaktoren, z.B. die letzte Blasensäule einer Kaskade (C), zurückgeführt werden. Der flüssig entnommene Produktstrom kann nach üblichen Methoden, z.B. durch Destillation aufgearbeitet und gereinigt werden.

In einer besonders bevorzugten Ausführungsform wird der flüssig zu entnehmende Produktstrom in 1 bis 5, bevorzugt 1 bis 3 nachgeschaltete Reaktoren geleitet, wobei dort eine weitere Reaktion im Sinne von Gleichung 2 und/oder 3 ablaufen kann. Diese Reaktoren sind beispielsweise weitere Blasensäulen, Rührkessel oder eine Reaktionsdestillation, die mit einer oder mehreren unter den Reaktionsbedingungen gasförmigen inerten Verbindungen begast werden (Leitung (9), gegebenenfalls über einen Vorwärmer/Verdampfer). In den Fig. 1 und 2 ist diese Fahrweise zur besseren Übersicht vereinfacht durch einen einzelnen Blasensäulenreaktor (D) veranschaulicht, wobei die erfindungsgemäße Fahrweise hierdurch nicht eingeschränkt werden soll.

In diesem Fall werden das aromatische Carbonat der Formel (I) bei (11) und das im Reaktor D entstandene flüchtige Reaktionsprodukt gemeinsam mit den gasförmigen Verbindungen bei (10') entnommen.

Der jeweils letzte Verweilzeitbehälter D kann gegebenenfalls ein nachgeschaltetes Abtriebsteil besitzen, mit dem leichtsiedende Produkte der Formel (IV) + (II) und/oder nicht umgesetzte Edukte der Formel (III) ganz oder teilweise in diesen Verweilzeitbehälter D zurückgeführt werdend Ebenso ist es gegebenenfalls von Vorteil, die am oberen Ende des ersten Verweilzeitbehälters D z.B. über (10') zu entnehmenden flüchtigen Reaktionsprodukte der Formel (IV) durch ein dort über die Leitung (10) aufgesetztes Verstärker- und/oder Dephlegmatorteil von höhersiedenden Produkten der Formel (I) oder Edukten der Formel (III) abzutrennen und diese in D zurückzuführen.

Als gasförmige Verbindungen im soeben genannten Sinne der Erfindung können z.B. überhitztes Phenol, inerte Gase allein, wie Stickstoff, Edelgase, Kohlendioxid, C₁-C₁₂-Alkane, cyclische Alkane, wie z.B. Cyclohexan, Dekalin, aromatische Kohlenwasserstoffe, wie Benzol, Toluol, Xylole, Cumol, Mesitylen, sowie Gemische aus Inertgasen oder Gemische aus Phenol mit Inertgas eingesetzt werden. In der bevorzugten Ausführungsform kommen leicht kondensierbare Verbindungen, wie Phenol, Toluol, Mesitylen, Dekalin, allein oder als Gemische zum Einsatz. Für den Fall, daß nur die erste Umesterungsstufe nach Gleichung (1) angestrebt wird, ist es jedoch durchaus möglich, in alle oder einzelne Blasensäulen sowie in die Verweilzeitbehälter Dialkylcarbonat, gegebenenfalls im Gemisch mit Inertgas, einzuführen. Ein solches Inertgas kann vorteilhafterweise wiederum ein Azeotropbildner für auszuschleusendes Alkanol sein.

Der am Blasensäulenreaktor oder gegebenenfalls am letzten Reaktor einer Blasensäulenkaskade nach der 1. Umesterungsstufe flüssig entnommene Produktstrom, der die Produkte der Formel (I) besonders im Sinne der Gleichung (1), in geringem Umfang auch im Sinne der Gleichungen (2) und (3) enthält, kann in einer weiteren besonderen Ausführungsform der Erfindung, gegebenenfalls nach Zwischenlagerung in geeigneten Behältern, anstelle des Edukts der Formel (III) zurück in den Blasensäulenreaktor oder gegebenenfalls in die 1. Blasensäule einer Blasensäulenkaskade eindosiert werden, um die 2. Umesterungsstufe im Sinne der Gleichung (2) oder eine Disproportionierung im Sinne der Gleichung (3) durchzuführen oder zu vervollständigen. Dies ist gegebenenfalls auch mehrfach möglich, wobei die Zuspeisung des zweiten Edukts der Formel (II) gegebenenfalls auch unterbleiben kann und durch unter Reaktionsbedingungen gasförmige inerte Verbindungen ersetzt wird. Zur kontinuierlichen Durchführung einer solchen Fahrweise sind z.B. entweder mindestens zwei Lagerbehälter oder ein Lagerbehälter mit mindestens zwei Kammern notwendig, wobei in der 1. Kammer das Produkt aus der laufenden Umsetzung eingespeist und aus der 2. Kammer das Edukt für die laufende Umsetzung entnommen wird. Wenn eine Kammer geleert bzw. eine Kammer gefüllt ist, wird die 2. Kammer zur Aufnahme des Produktes aus dem Blasensäulenreaktor bzw. aus dem letzten Reaktor einer Blasensäulenkaskade und die 1. Kammer zur Einspeisung des Eduktes in den Blasensäulenreaktor bzw. in die Blasensäulekaskade benutzt.

Alternativ kann in einer weiteren Ausführungsform eine weitere Behandlung des flüssigen Reaktionsproduktes aus der 1. Umesterungsstufe, wie z.B. in Fig. 1 und 2 der Ablauf von Leitung (4) nach Reaktor (C), in einer mehrstufigen Destillationsapparatur im Sinne von EP 0 461 274 erfolgen, wobei dort eine weitere Reaktion im Sinne von Gleichung (2) und/oder (3) ablaufen kann.

In einer weiteren Variante ist der Verweilzeitbehälter D in Form einer Destillationsapparatur ausgebildet, die im Sinne einer "Reaktionsdestillation" betrieben wird, das heißt, daß simultan zur ablaufenden Reaktion eine Destillation der beteiligten Stoffe durchgeführt wird.

Die für eine "Reaktionsdestillation" im Sinne der Erfindung wesentlichen Merkmale sind die folgenden: Das noch nicht umgesetzte Alkylarylcarbonat-Zwischenprodukt aus der 1. Umesterungsstufe wird durch einen speziell gewählten Temperaturgradienten in der Destillationsapparatur weitgehend daran gehindert, den Reaktionsteil des Reaktors nach oben oder nach unten zu verlassen. Die leichtflüchtigen Reaktionsprodukte der Formel (IV) werden am Kopf der kolonne, das schwerflüchtige Reaktionsprodukt, hier das Diarylcarbonat (2. Umesterungsstufe), wird am Fuß der Kolonne entnommen. Gegebenenfalls vorhandenes, überschüssiges Phenol kann mit den Diarylcarbonat-Endprodukten am Fuß der Destillationsapparatur oder mit den Leichtsiederprodukten am Kopf der Apparatur entnommen werden.

Der als "Reaktionskolonne" bezeichnete Reaktor besteht aus einem kolonnenartigen Rohr dem ein Temperaturprofil angelegt wird, das von oben nach unten gesehen ansteigend einen Temperaturbereich von 60 bis 320°C, bevorzugt 65 bis 305°C und besonders bevorzugt von 65 bis 250°C, umfaßt. Zur Einstellung der Temperaturgradienten in den einzelnen Abschnitten des kolonnenartigen Reaktors können diese Abschnitte mit einer Isolierung bzw. einer Thermostatisierung versehen werden. Die Thermostatisierung kann hierbei je nach Bedarf eine Heizung oder eine Kühlung darstellen. Die Reaktionskolonne kann in verschiedenen Abschnitten ihrer Gesamtlänge, entsprechend den Gas- und Flüssigbelastungen und den benötigten Verweilzeiten aufgeweitet oder verengt sein.

Für den mittleren Teil der Reaktionskolonne, den Reaktionsbereich, sind feste Einbauten bevorzugt, für die Teile, in denen Trennungen stattfinden, dagegen Füllkörper und feste Packungen.

Am unteren Ende der Reaktionskolonne sind ein oder mehrere, gegebenenfalls durch adiabatisch isolierte Kolonnenteile getrennte Verdampfer angeordnet. Diese Verdampfer können innerhalb oder außerhalb der Kolonne angeordnet sein. In einer technischen Ausführung werden in der Technik übliche Apparate wie z.B. Umlaufverdampfer, Fallfilmverdampfer und Wendelrohrverdampfer verwendet.

Oberhalb der Verdampferzone, in dem als "Reaktionszone" bezeichneten mittleren Bereich, werden bevorzugt feste Einbauten oder beispielsweise Glockenböden benutzt. Die theoretische Bodenzahl in diesem Bereich beträgt 1 bis 50, bevorzugt 1 bis 25 und besonders 1 bis 15.

Wiederum oberhalb dieses Bereichs ist die Kolonne mit weiteren, im besonderen Maße für destillative Stofftrennungen geeigneten Füllkörpern, Packungen oder Einbauten ausgestattet. Am oberen Ende der Kolonne ist bevorzugt ein Verstärkerteil angeordnet, mit dem ein gezielter Rücklauf der Kolonne einstellbar ist.

Die Reaktionskolonne wird so betrieben, daß man oberhalb der "Reaktionszone" den aus dem Blasensäulenreaktor oder der Blasensäulenkaskade flüssig entnommenen Produktstrom aus der 1. Umesterungsstufe flüssig eindosiert. Dieser Strom durchläuft die "Reaktionszone" und wird dort teilweise in Diarylcarbonat nach Gleichung (2) und (3) verwandelt, und die noch nicht umgesetzten Reaktanden werden mit Hilfe der beschriebenen Verdampfer gasförmig zurück in die Reaktionszone und die oberen Teile der Kolonne transportiert. Diese kondensieren dort und setzen sich erneut zum Diarylcarbonat-Endprodukt um. Das Diarylcarbonat-Endprodukt wird als höchst siedende Reaktionskomponente im Sumpfbereich der Kolonne angereichert und dort zusammen mit gegebenenfalls homogen gelöstem Katalysator und geringen Mengen Alkylarylcarbonat und aromatischer Hydroxyverbindung ausgespeist.

Die leichtflüchtigen Reaktionsprodukte der Formel (IV) werden am Kopf der Kolonne entnommen. Die im Überschuß vorhandenen oder nicht umgesetzten Phenole der Formel (III) können am Fuß der Kolonne mit dem DiarylcarbonatEndprodukt der Formel (I) oder in einer bevorzugten Fahrweise mit den Leichtsiederprodukten am Kopf der Kolonne ausgespeist werden.

In einer weiteren Verfahrensweise kann der flüssig zu entnehmende Produktstrom in 1 bis 5, bevorzugt 1 bis 3 nachgeschaltete gegebenenfalls gerührte oder mit Inertgas begaste Verweilzeitbehälter D geleitet werden, wobei dort weitere Reaktionen im Sinne von Gleichung 2 und/oder Gleichung 3 ablaufen können. In diesem Falle werden das aromatische Carbonat der Formel (I) bei (11) und in D entstandene flüchtige Reaktionsprodukte bei (10) bzw. (10') entnommen.

Zur Vermischung der Reaktionskomponenten sind die erfindungsgemäß zu verwendenden Rührbehälter mit dafür brauchbaren Rührwerkzeugen ausgestattet. Solche Rührer sind dem Fachmann bekannt. Es seien beispielhaft genannt: Scheiben-, Impeller-, Propeller-, Schaufel-, MIG- und Intermig-Rührer, Rohrrührer und andere Hohlrührertypen. Bevorzugte Rührer sind solche, die eine effektive Vermischung von Gasen und Flüssigkeiten erlauben, beispielsweise Hohlrührer, wie Rohrrührer und Dreikantrührer, Propellerrührer, Turbinenrührer etc.

Zur besseren Vermischung können die Rührbehälter bevorzugt mit Strömungsbrecher-Einbauten versehen sein. Diese Strömungsbrecher können gleichzeitig thermostatisierbar zum Einbringen oder zum Abführen von Wärme aus dem Reaktor ausgelegt sein.

Bevorzugt werden solche Arbeitsweisen und Ausführungsformen der Erfindung benutzt, bei denen zusätzliche Verweilzeitbehälter in Form von Säulen oder Rührkesseln benutzt werden.

Mögliche apparative Ausführungsformen zur Durchführung des erfindungsgemäßen Verfahrens sind folgende, wobei die Aufzählung keineswegs erschöpfend ist:
- eine Blasensäule,
- eine Blasensäule mit einem Verweilzeitbehälter in Form eines Rührkessels und/oder einer Destillationskolonne,
- eine Blasensäule mit mehreren Verweilzeitbehältern in Form von Rührkesseln und/oder Destillationskolonnen,
- eine Kaskade aus zwei oder mehr Blasensäulen,
- eine Blasensäulenkaskade aus zwei oder mehr Blasensäulen mit einem Verweilzeitbehälter in Form eines Rührkessels oder einer Destillationskolonne,
- eine Kaskade aus zwei oder mehr Blasensäulen mit mehreren Verweilzeitbehältern in Form von Rührkesseln und/oder Destillationskolonnen,
wobei in allen Fällen Blasensäulen ohne oder mit Einbauten der genannten Art verwendet werden können.

Die für die Reaktion notwendige Reaktionswärme kann mit den Edukten eingebracht werden. Es ist jedoch bevorzugt, zusätzliche Energie in den Reaktor beispielsweise über eine Mantelbeheizung und/oder durch innenliegende Beheizungselemente einzubringen.

Die weitere Aufarbeitung der über Leitung (11) entnommenen flüssigen Reaktionsprodukte der Formel (I), die überschüssige phenolische Verbindung (III) und gegebenenfalls noch einen homogenen gelösten Katalysator enthalten können, kann nach üblichen Methoden, z.B. durch Destillation erfolgen.

In einer bevorzugten Ausführungsform, wenn als Katalysator eine Titan-Verbindung, beispielsweise Titantetraphenolat verwendet wird, kann dieser vor der destillativen Aufarbeitung des flüssigen Reaktionsproduktes durch Kristallisation und anschließende Filtration oder Sedimentation vom Reaktionsprodukt der 2. Umesterungsstufe abgetrennt werden.

Zur Abtrennung wird hierzu das flüssige Reaktionsgemisch auf eine Temperatur von 40 bis 120°C, bevorzugt 50 bis 100°C besonders bevorzugt 60 bis 90°C abgekühlt, wobei dieses Gemisch flüssig bleiben muß. Man kann dann den ausgefallenen, Titan enthaltenden Niederschlag abtrennen. Das verbleibende Reaktionsgemisch enthält restliche Titanmengen von weniger als 100 ppm. Der so abgetrennte Katalysator kann gegebenenfalls ohne weitere Reinigung in den Prozeß zurückgeführt werden.

Durch die erfindungsgemäße Abkühlung des Reaktionsgemisches und die Abtrennung des ausgefallenen, Titan enthaltenden Niederschlages erhält man in einer überraschend einfachen Operation ein Reaktionsgemisch, das man sowohl durch Kristallisation als auch durch Destillation unter an sich üblichen Bedingungen zur Gewinnung des aromatischen Carbonats aufarbeiten kann, ohne Ausbeuteverluste befürchten zu müssen. Besondere Reaktionsbedingungen und besondere Vorsichtsmaßnahmen, die durch die Anwesenheit des Katalysators erforderlich wären, sind daher nicht mehr erforderlich.

Die Abtrennung des Titankatalysators kann gegebenenfalls auch bereits nach der ersten Umesterungsstufe (nach Reaktor (C) in Fig. 1 und 2) erfolgen, wenn beispielsweise ein Alkylarylcarbonat angestrebt wird oder für die 2. Umesterungsstufe ein anderer Katalysator vorgesehen ist.

Die zu verwendenden und als solche bekannten Umesterungskatalysatoren werden bevorzugt zusammen mit den flüssig zu dosierenden Edukten der Formel (III) in gelöster oder suspendierter Form in den Blasensäulenreaktor oder die Blasensäulenkaskade eingebracht. Alternativ kann der Katalysator auch separat oder gelöst bzw. suspendiert in einer geringen Menge des Eduktes der Formel (III) oder in einem systemfremden, geeigneten, inerten Lösungsmittel s.o. eindosiert werden. Im Falle der Verwendung heterogener Katalysatoren können diese auch im Blasensäulenreaktor oder in der Blasensäulenkaskade direkt ortsfest eingesetzt werden.

Durch geeignete Filtriervorrichtung muß dabei das Austragen der Katalysatoren verhindert werden.

Wichtig ist, daß bei einer Kaskaden-Blasensäule auf mindestens 2 Verteilungsorganen bzw. in einer Blasensäulenkaskade in mindestens 2 Blasensäulen ein Katalysator vorhanden ist.

Im Falle der Verwendung von nicht ortsfesten Katalysatoren ist es möglich, die Katalysatoren nach teilweiser oder vollständiger Abtrennung von den Produkten oder Edukten wieder wie oben beschrieben in den Reaktionsprozeß zurückzuführen, wobei gegebenenfalls ein der desaktivierten Katalysatormenge entsprechender Anteil des Katalysators abgetrennt und durch frischen Katalysator ersetzt wird.

Das erfindungsgemäße Verfahren wird bei Temperaturen in der Flüssigphase von 80 bis 350°C, bevorzugt bei 100 bis 250°C und besonders bevorzugt bei Temperaturen von 120 bis 240°C durchgeführt. Dabei soll die Flüssigphasentemperatur in den Blasensäulenreaktoren nicht über der Verdampfungstemperatur der eingesetzten phenolischen Verbindung der Formel (III) bzw. der phenolischen Lösung liegen. Es kann deshalb von Vorteil sein, die erfindungsgemäße Umesterung im Bereich der Blasensäulenreaktoren nicht nur bei Normaldruck, sondern auch bei erhöhtem oder erniedrigtem Druck im Bereich von 10 mbar bis 20 bar durchzuführen. Ein bevorzugter Druckbereich liegt zwischen 0,05 und 15 bar, ein besonders bevorzugter Druckbereich liegt zwischen 0,08 und 13 bar. Hierbei kann es günstig sein, die einzelnen Reaktoren einer Kaskade bei jeweils unterschiedlichen Drücken zu betreiben. Mit den Drücken kann gegebenenfalls die Temperatur in den einzelnen Blasensäulenreaktoren einer Kaskade variiert werden. In einer bevorzugten Ausführungsform können z.B. sowohl Druck als auch Temperatur von dem 1. zum letzten Blasensäulenreaktor fallen.

Katalysatoren, die für das erfindungsgemäße Verfahren in Frage kommen und die für alle Phasen des erfindungsgemäßen Verfahrens gleich sein können, sind in der Literatur bekannt. Solche Katalysatoren sind beispielsweise Hydride, Oxide, Hydroxide, Alkoholate, Amide oder Salze von (Erd)Alkalimetallen, wie Lithium, Natrium, Kalium, Rubidium, Cäsium, Magnesium und Calcium, bevorzugt von Lithium, Natrium, Kalium, Magnesium und Calcium, besonders bevorzugt von Lithium, Natrium und Kalium (US-3 642 858, US-3 803 201, EP 1082). Für den Fall des Einsatzes der Alkoholate können diese erfindungsgemäß auch in situ durch Einsatz der elementaren Alkalimetalle und des erfindungsgemäßen umzusetzenden Alkohols gebildet werden. Salze der (Erd)Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff- oder Iodwasserstoffsäure, Salpetersäure, Schwefelsäure, Fluorwasserstoffsäure, Phosphorsäure, Blausäure, Rhodanwasserstoff, Borsäure, Zinnsäure, C₁-C₄-Stannonsäuren oder Antimonsäuren. In bevorzugter Weise kommen als Verbindungen der (Erd)Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt.

Solche (Erd)Alkalimetallverbindungen (gegebenenfalls in situ gebildet aus den freien Alkalimetallen) werden in Mengen von 0,001 bis 2 Gew.-%, bevorzugt 0,005 bis 0,9 Gew.-%, besonders bevorzugt 0,01 bis 0,5 Gew.-%, bezogen auf das umzusetzende Reaktionsgemisch, eingesetzt.

Weitere erfindungsgemäß einsetzbare Katalysatoren sind Lewis-saure Metallverbindungen wie AlX₃, TiX₃, UX₄, TiX₄, VOX₃, VX₅, ZnX₂, FeX₃ und SnX₄, worin X für Halogen, Acetoxy oder Aryloxy steht (DE-OS 2 528 412, 2 552 907), beispielsweise Titantetrachlorid, Titantetraphenoxid, Titantetraethoxid, Titantetraisopropylat, Titantetradodecylat, Zinntetraisooctylat und Aluminiumtriisopropylat, weiterhin zinnorganische Verbindungen der allgemeinen Formel (R⁴)₄₋ₓ-Sn(Y)ₓ, in der Y für einen Rest OCOR⁵, OH oder OR steht, wobei R⁵ C₁-C₁₂-Alkyl, C₆-C₁₂-Aryl oder C₇-C₁₃-Alkylaryl bedeutet und R⁴ unabhängig von R⁵ den Bedeutungsumfang von R⁵ annehmen kann und x eine ganze Zahl von 1 bis 3 bedeutet, Dialkylzinnverbindungen mit 1 bis 12 C-Atomen im Alkylrest oder Bis(trialkylzinn)verbindungen, beispielsweise Trimethylzinnacetat, Triethylzinnbenzoat, Tributylzinnacetat, Triphenylzinnacetat, Dibutylzinndiacetat, Dibutylzinndilaurat, Dioctylzinndilaurat, Dibutylzinnadipinat, Dibutyldimethoxyzinn, Dimethylzinnglykolat, Dibutyldiethoxyzinn, Triethylzinnhydroxid, Hexaethylstannoxan, Hexabutylstannoxan, Dibutylzinnoxid, Dioctylzinnoxid, Butylzinntriisooctylat, Octylzinntriisooctylat, Butylstannonsäure und Octylstannonsäure in Mengen von 0,001 bis 20 Gew.-% (EP 879, EP 880, EP 39 452, DE-OS 3 445 555, JP 79/62 023), polymere Zinnverbindungen der Formel -[-R⁴,R⁵Sn-O-]-, beispielsweise Poly[oxy(dibutylstannylen)], Poly[oxy(dioctylstannylen)], Poly[oxy(butylphenylstannylen)] und Poly[oxy(diphenylstannylen)] (DE-OS3 445 552), polymere Hydroxystannoxane der Formel -[R⁴Sn(OH)-O-]-, beispielsweise Poly(ethylhydroxystannoxan), Poly(butylhydroxystannoxan), Poly(octylhydroxystannoxan), Poly(undecylhydroxystannoxan) und Poly(dodecylhydroxystannoxan) in Mengen von 0,001 bis 20 Gew.-%, bevorzugt von 0,005 bis 5 Gew.-%, bezogen auf Kohlensäurediester (DE 4 006 520). Weitere erfindungsgemäß einsetzbare Zinnverbindungen sind Sn(II)oxid oder besitzen die Formel

X¹-Sn(R⁴)₂-O-Sn(R⁴)₂-X² (IX)

worin
X¹ und X² unabhängig voneinander OH, SCN, OR⁴, OCOR⁴ oder Halogen und
R⁴ Alkyl, Aryl bedeutet (EP 338 760).

Als weitere erfindungsgemäß einsetzbare Katalysatoren kommen Bleiverbindungen, gegebenenfalls zusammen mit Triorganophosphanen, einer Chelatverbindung oder einem Alkalimetallhalogenid, beispielsweise Pb(OH)₂·2PbCO, Pb(OCO-CH₃)₂, Pb(OCO-CH₃)₂·2LiCl, Pb(OCO-CH₃)₂·2PPh₃ in Mengen von 0,001 bis 1, bevorzugt von 0,005 bis 0,25 mol pro Mol Carbonat (JP 57/176 932, JP 01/093 580), andere Blei(II)- und Blei(IV)-verbindungen, wie PbO, PbO₂, Mennige, Plumbite (PbO₂²⁻) und Plumbate (PbO₃²⁻) (JP 01/093 560), Eisen(III)acetat (JP 61/172 852), weiterhin Kupfersalze und/oder Metallkomplexe, beispielsweise von Alkali-, Zink-, Titan- und Eisen (JP 89/005 588), Kombinationen aus Lewis-Säuren und Protonensäuren (DE-OS 3 445 553) oder Elementverbindungen von Sc, Cr, Mo, W, Mn, Au, Ga, In, Bi, Te und Lanthaniden (EP 338 760) in Frage.

Weiterhin sind im erfindungsgemäßen Verfahren heterogene Katalysatorsysteme einsetzbar. Solche sind beispielsweise Mischoxide aus Silicium und Titan, die durch gemeinsame Hydrolyse von Silicium- und Titanhalogeniden herstellbar sind (JP 54/125 617) und Titandioxide mit hoher BET-Oberfläche > 20 m²/g (DE-OS 4 036 594).

Bevorzugt im erfindungsgemäßen Verfahren einsetzbare Katalysatoren sind Zinn-, Titan- und Zirkoniumverbindungen und die obengenannten Alkali- und Erdalkaliverbindungen, besonders bevorzugt einsetzbare Katalysatoren sind Organozinnverbindungen und Titantetraalkoholate und -phenolate.

Die einzusetzenden Katalysatormengen betragen 0,01 bis 10 mol-%, bevorzugt 0,05 bis 5 mol-% und besonders bevorzugt 0,01 bis 2 mol-%, bezogen auf die eingesetzte Phenol- oder Alkylarylcarbonatkomponente, und können sich teilweise von den in der Literatur genannten Mengen unterscheiden.

Die folgenden Beispiele sollen die vorliegende Erfindung konkret erläutern, wobei sie nicht auf diese Beispiele beschränkt sein soll.

### Beispiele

### Beispiel 1

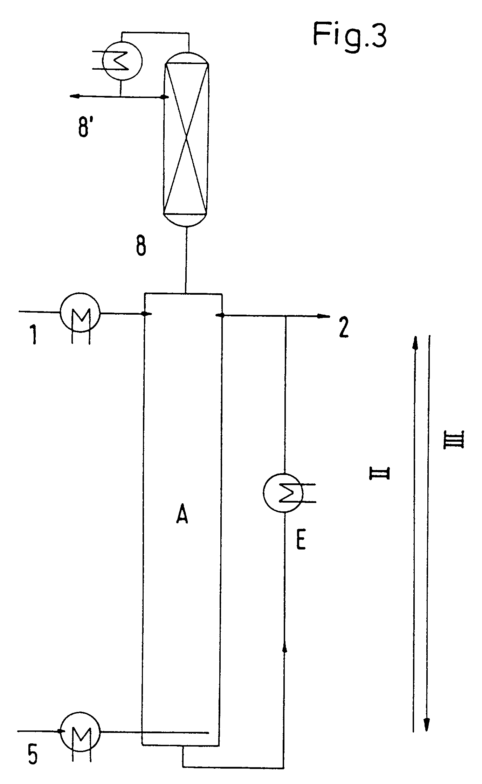

(Apparatur siehe Figur 3; sie stellt eine Ausführung mit nur einer Blasensäule dar. Die Bezugszeichen haben die oben gegebene Bedeutung, wobei über Leitung (2) nicht wie in Fig. 1 und 2 in die nächste Blasensäule dosiert wird, sondern als das Reaktionsgemisch entnommen wird).

Für dieses Beispiel wurde eine mit einem Heizmantel versehene und mit einem Öl-Thermostaten beheizbare Blasensäule (l = 60 cm, d = 4,5 cm mit 10 Lochplatten zur Dispergierung der Gasphase) mit 950 ml Innenvolumen eingesetzt. Die Dosierung der Flüssigphase erfolgte am oberen Ende der Blasensäule über eine beheizte Leitung und die Entnahme am unteren Ende über einen in der Höhe verstellbaren beheizten Siphon. Die Gasphase wurde am unteren Ende der Blasensäule über eine Glassinterplatte eingespeist und am Kopf über eine 30 cm lange mit Raschigringen gefüllte Kolonne mit aufgesetztem Kolonnenkopf, der die Einstellung eines Rücklaufs auf den Blasensäulenreaktor erlaubte, entnommen.

Die Blasensäule wurde mit 850 ml Phenol gefüllt und der Reaktormantel mit Öl auf 180°C thermostatisiert. Über eine beheizte Pumpe wurden 500 g/h einer Mischung aus 97,8 Gew.-% Phenol und 2,2 Gew.-% Titantetraphenolat (Flüssigphase) am oberen Ende des Blasensäulenreaktors kontinuierlich zudosiert und gleichzeitig 500 g/h Dimethylcarbonat (DMC), das kontinuierlich in einem elektrisch beheizten Rohr verdampft wurde, am unteren Ende zudosiert. Nach 4 h befand sich die Reaktion im Gleichgewicht, d.h. die Zusammensetzung der Gas- und Flüssigphase änderte sich nicht mehr. Am Reaktoraustritt wurden über den Siphon 557 g/h Produktgemisch mit 65,7 g/h Methylphenylcarbonat (MPC) und 13,5 g/h Diphenylcarbonat (DPC) entnommen. Der Rest zu 100 % waren Phenol, wenig Dimethylcarbonat und Katalysator. Am oberen Ende der Blasensäule wurde über die aufgesetzte Kolonne ein Produktgemisch aus Methanol und DMC entnommen. Daraus ergibt sich eine Raum-Zeit-Ausbeute für die MPC-und DPC-Bildung von 83,0 g/l h. Die Selektivität bezüglich der Bildung aromatischer Carbonate war > 99,9 %.

### Beispiel 2

In der in Beispiel 1 beschriebenen Apparatur und unter den dort angegebenen Reaktionsbedingungen wurden 750 g/h eines Gemisches aus 98,6 Gew.-% Phenol und 1,4 Gew.-% Octylstannonsäure am oberen Ende der Blasensäule und 750 g/h DMC am unteren Ende der Blasensäule kontinuierlich eingespeist. Nach ca. 3 h befand sich die Reaktion im Gleichgewicht. Kontinuierlich wurden 793 g/h flüssiges Produktgemisch mit 105,6 g MPC und 23 g/h DPC und am oberen Ende der Blasensäule ein Gemisch aus Methanol und DMC entnommen. Dies entspricht einer Raum-Zeit-Ausbeute für MPC und DPC von 135 g/l h. Die Selektivität war > 99,9 %.

### Beispiel 3

Für dieses Beispiel wurde eine Blasensäule von 150 cm Länge und 2,8 cm Durchmesser (923 ml Innenvolumen) und mit einer Füllung aus 3x3 mm V4A-Maschendrahtwendeln eingesetzt. Der Reaktormantel wurde auf 180°C aufgeheizt und die Blasensäule mit 600 ml Phenol gefüllt. Analog zu den Beispielen 1 und 2 wurden 250 g/h Phenol mit 1,4 Gew.-% Octylstannonsäure und 250 g/h DMC dosiert. Nach ca. 3 h befand sich die Reaktion im Gleichgewicht und es wurden 270 g/h flüssiges Produkt mit 51 g MPC und 10,5 g DPC über den Siphon entnommen. Dies entspricht einer Raum-Zeit-Ausbeute von 66,6 g/l h. Die Selektivität betrug auch hier 99,9 %.

### Beispiel 4

Das Beispiel 2 wurde mit den dort angegebenen Reaktionsbedingungen und Eduktströmen wiederholt. Zusätzlich erfolgte die kontinuierliche Einleitung der am Reaktor A (Figur 3) entnommenen Flüssigphase am oberen Ende eines weiteren Blasensäulenreaktors (Reaktor D in Figur 1 und 2). Dieser Blasensäulenreaktor (baugleich mit Reaktor A) war ebenfalls über eine Mantelheizung (mit Öl auf 180°C thermostatisiert) versehen.

Gleichzeitig mit der Flüssigphase wurde ein Stickstoffstrom von 100 Nl/h in einem elektrisch beheizten Rohr vorgeheizt und am unteren Ende der weiteren Blasensäule zudosiert. Nach 6 h befand sich die Reaktion im Gleichgewicht.

Am unteren Ende der zweiten Blasensäule liefen über einen Austrag kontinuierlich 767,3 g/h flüssiges Produktgemisch mit 21,1 g MPC, 85,4 g DPC und 660,8 g Phenol ab. In einer Tiefkühlfalle kondensierten aus dem Stickstoffstrom stündlich 25 g einer Mischung aus DMC und Methanol. Dies entspricht einer Raum-Zeit-Ausbeute für MPC und DPC, bezogen auf das gesamte Reaktionsvolumen der beiden Reaktoren, von 56,1 g/lh.

### Vergleichsbeispiel

Ein beheizter Rührbehalter mit 1 l Innenvolumen, der mit einer 1 m langen, mit 4 x 4 mm Glasringen gefüllten Kolonne ausgestattet war, wurde mit 500 g Phenol und 11 g Titantetraphenolat gefüllt. Nach der Aufheizung des Behälterinhalts auf 175°C bis 180°C erfolgte die Zudosierung des DMC derart, daß die Innentemperatur nicht abfiel. Innerhalb von 4 h wurden 78 g DMC zudosiert. Gleichzeitig destillierten über die Kolonne 49,1 g eines Gemisches aus DMC und Methanol ab. Das Sumpfprodukt bestand nach dieser Zeit aus 451,4 g Phenol, 58,5 g MPC, 13 g DPC, 2,2 g Nebenprodukte und 3,7 g DMC. Daraus ergibt sich ein Phenolumsatz von 9,7 % und eine Selektivität von 97,9 %, bezogen auf umgesetztes Phenol. Die Raum-Zeit-Ausbeute für die Bildung der aromatischen Carbonate war somit 8,94 g/lh.

## Patentansprüche

1. Verfahren zur Herstellung eines aromatischen Carbonats der Formel
R¹-O-CO-O-R² (I)
in der
R² Phenyl oder Naphthyl sowie ein- bis dreifach durch geradkettiges oder verzweigtes C₁-C₄-Alkyl, geradkettiges oder verzweigtes C₁-C₄-Alkoxy, Cyano und/oder Halogen substituiertes Phenyl bzw. Naphthyl bedeutet, und
R¹ unabhängig von R² den Bedeutungsumfang von R² annimmt oder geradkettiges oder verzweigtes C₁-C₆-Alkyl bedeutet,
durch katalysierte Umsetzung von je 0,1-10 mol, bevorzugt 0,2-5 Mol, besonders bevorzugt 0,5-3 mol eines organischen Carbonats mit mindestens einer aliphatischen Estergruppe der Formel
R¹-OCOO-R³ (II)
in der
R³ geradkettiges oder verzweigtes C₁-C₆-Alkyl bedeutet und
R¹ den obigen Bedeutungsumfang hat,
bevorzugt eines symmetrischen Dialkylcarbonats der Formel
R³-O-CO-O-R³ (VI),
in der
R³ den obigen Bedeutungsumfang hat,
mit je 1 mol einer phenolischen Verbindung der Formel
R²-OX (III)
in der
R² den obigen Bedeutungsumfang hat und
X für Wasserstoff oder für -CO-O-C₁-C₆-Alkyl mit geradkettiger oder verzweigter Alkylgruppe steht,
bevorzugt mit je 1 Mol einer phenolischen Verbindung der Formel
R¹²-OH (V),
in der
R¹² Phenyl oder einfach durch C₁-C₄-Alkyl, C₁-C₄-Alkoxy oder Chlor substituiertes Phenyl bedeutet,
in Gegenwart eines an sich bekannten Umesterungskatalysators bei 80-350°C und 10 mbar bis 20 bar, dadurch gekennzeichnet, daß die Umsetzung in einem Blasensäulenreaktor oder einer Kaskade aus mindestens zwei Blasensäulen so durchgeführt wird, daß die phenolische Verbindung der Formel (III) in flüssiger Form in die erste Blasensäule und das organische Carbonat der Formel (II) flüssig oder gasförmig gleichzeitig in jede einzelne, bevorzugt aber nur in die letzte Blasensäule eindosiert werden, wobei bei flüssiger Eindosierung eine Verdampfung von (II) in der Blasensäule erfolgt, und aus der letzten Blasensäule die Reaktionsprodukte der Formel (I) in flüssiger Form und gleichzeitig am oberen Ende jeder einzelnen Blasensäule, bevorzugt am oberen Ende der ersten Blasensäule, gasförmig die Produkte der Formel
R³-OX (IV)
in der R³ und X die genannte Bedeutung haben, entnommen werden.

2. Verfahren nach Anspruch 1, dadurch gekennzeichnet, daß die Umsetzung in 1 bis 18, bevorzugt in 2 bis 12 Blasensäulen durchgeführt wird.

3. Verfahren nach Anspruch 1, dadurch gekennzeichnet, daß die Umsetzung in mindestens zwei hintereinandergeschalteten Blasensäulenreaktoren so durchgeführt wird, daß das organische Carbonat der Formel (II) in die erste Blasensäule eindosiert wird und aus der letzten Blasensäule das aromatische Carbonat der Formel (I) in flüssiger Form und am oberen Ende der ersten Blasensäule das Produkt der Formel (IV) entnommen wird.

4. Verfahren nach Anspruch 1, dadurch gekennzeichnet, daß bei 100 -250°C, bevorzugt bei 120 bis 240°C gearbeitet wird, wobei im Falle einer Blasensäulenkaskade die Temperaturen in den Blasensäulen gleich oder verschieden sind.

5. Verfahren nach Anspruch 1, dadurch gekennzeichnet, daß im Druckbereich von 0,05 bis 15 bar, bevorzugt 0,08 bis 13 bar gearbeitet wird, wobei im Falle einer Blasensäulenkaskade die Drücke in den einzelnen Blasensäulen gleich oder verschieden sind.

6. Verfahren nach Anspruch 1, dadurch gekennzeichnet, daß im Falle einer Blasensäulenkaskade sowohl der Druck als auch die Temperatur von der ersten zur letzten Blasensäule fallen.

7. Verfahren nach Anspruch 1, dadurch gekennzeichnet, daß Blasensäulen mit Füllkörpern, geordneten Packungen oder Lochböden eingesetzt werden.

8. Verfahren nach Anspruch 1, dadurch gekennzeichnet, daß eine Blasensäule oder eine Blasensäulen-Kaskade mit einem oder mehreren nachgeschalteten Verweilzeitbehälter(n) kombiniert wird.

9. Verfahren nach Anspruch 1, dadurch gekennzeichnet, daß das organische Carbonat (II) im Gemisch mit 0-5 Gew.-%, bevorzugt 0,1-3 Gew.-%, besonders beborzugt 0,15-2 Gew.-%, bezogen auf das Gewicht von (II), an zugrundeliegendem Alkohol R³-OH eingesetzt wird.

10. Verfahren nach Anspruch 1, dadurch gekennzeichnet, daß zusätzlich zu den Edukten an beliebiger Stelle der Blasensäule oder Blasensäulenkaskade gemeinsam mit dem Carbonat der Formel (II) oder separat davon ein inertes, im Reaktionsgemisch verdampfendes Lösungsmittel oder ein inertes Gas eingespeist wird, das bevorzugt ein Azetrop mit dem Produkt der Formel (IV) bildet.

## Claims

1. Process for the preparation of an aromatic carbonate of the formula
R¹-O-CO-O-R² (I)
in which
R² denotes phenyl or naphthyl each of which may be monosubstituted to trisubstituted by straight-chain or branched C₁-C₄-alkyl, straight-chain or branched C₁-C₄-alkoxy, cyano and/or halogen, and
R¹, independently of R², assumes the range of meanings of R² or denotes straight-chain or branched C₁-C₆-alkyl,
by catalysed reaction of in each case 0.1-10 mol, preferably 0.2-5 mol, particularly preferably 0.5-3 mol, of an organic carbonate having at least one aliphatic ester group of the formula
R¹-OCOO-R³ (II)
in which
R³ denotes straight-chain or branched C₁-C₆-alkyl and
R¹ has the above range of meanings,
preferably of a symmetrical dialkyl carbonate of the formula
R³-O-CO-O-R³ (VI)
in which
R³ has the above range of meanings,
with in each case 1 mol of a phenolic compound of the formula
R²-OX (III)
in which
R² has the above range of meanings and
X represents hydrogen or -CO-O-C₁-C₆-alkyl having a straight-chain or branched alkyl group,
preferably with in each case 1 mol of a phenolic compound of the formula
R¹²-OH (V)
in which
R¹² denotes phenyl or phenyl monosubstituted by C₁-C₄-alkyl, C₁-C₄-alkoxy or chlorine,
in the presence of a transesterification catalyst known per se at 80-350°C and 10 mbar to 20 bar, characterized in that the reaction is carried out in a bubble column reactor or a cascade of at least two bubble columns in such a way that the phenolic compound of the formula (III) is metered in in livid form into the first bubble column and the organic carbonate of the formula (II) is metered in in the liquid or gaseous state simultaneously into each individual bubble column, but preferably only into the last bubble column, in the case of liquid metering, an evaporation of (II) in the bubble column proceeding, and the reaction products of the formula (I) are taken off from the last bubble column in liquid form and simultaneously at the upper end of each individual bubble column, preferably at the upper end of the first bubble column, the products of the formula
R³-OX (IV)
in which R³ and X have the meaning mentioned, are taken off in gaseous form.

2. Process according to Claim 1, characterized in that the reaction is carried out in 1 to 18, preferably in 2 to 12, bubble columns.

3. Process according to Claim 1, characterized in that the reaction is carried out in at least two sequentially-connected bubble column reactors in such a way that the organic carbonate of the formula (II) is metered into the first bubble column and the aromatic carbonate of the formula (I) is taken off in liquid form from the last bubble column and the product of the formula (IV) is taken off at the upper end of the first bubble column.

4. Process according to Claim 1, characterized in that a temperature of 100-250°C, preferably 120 to 240°C, is employed, in the case of a bubble column cascade, the temperatures in the bubble columns being identical or different.

5. Process according to Claim 1, characterized in that a pressure range of 0.05 to 15 bar, preferably 0.08 to 13 bar, is employed, in the case of a bubble column cascade, the pressures in the individual bubble columns being identical or different.

6. Process according to Claim 1, characterized in that in the case of a bubble column cascade, both the pressure and the temperature decrease from the first to the last bubble column.

7. Process according to Claim 1, characterized in that bubble columns having loose packings, arranged packings or perforated trays are used.

8. Process according to Claim 1, characterized in that a bubble column or a bubble column cascade is combined with one or more downstream residence time vessels.

9. Process according to Claim 1, characterized in that the organic carbonate (II) is used in a mixture with 0-5% by weight, preferably 0.1-3% by weight, particularly preferably 0.15-2% by weight, based on the weight of (II), of the underlying alcohol R³-OH.

10. Process according to Claim 1, characterized in that, additionally to the starting materials, an inert solvent evaporating in the reaction mixture or an inert gas is fed in together with the carbonate of the formula (II) or separately therefrom at any desired position of the bubble column or bubble column cascade, which solvent or gas preferably forms an azeotrope with the product of the formula (IV).

## Revendications

1. Procédé de préparation d'un carbonate aromatique de formule
R¹-O-CO-O-R² (I)
dans laquelle
R² représente un groupe phényle ou naphtyle ou un groupe phényle ou naphtyle portant un à trois substituants alkyles à chaîne droite ou ramifiée en C₁-C₄, alcoxy à chaîne droite ou ramifiée en C₁-C₄, cyano et/ou halogéno, et
R¹ indépendamment de R², a les mêmes significations que celui-ci ou représente un groupe alkyle à chaîne droite ou ramifiée en C₁-C₆,
par réaction catalysée de 0,1 à 10 mol, de préférence de 0,2 à 5 mol et plus spécialement de 0,5 à 3 mol d'un carbonate organique contenant au moins un groupe ester aliphatique et répondant à la formule
R¹-O-CO-O-R³ (II)
dans laquelle
R³ représente un groupe alkyle à chaîne droite ou ramifiée en C₁-C₆ et
R¹ a les significations indiquées ci-dessus,
de préférence un carbonate de dialkyle symétrique de formule
R³-O-CO-O-R³ (VI),
dans laquelle
R³ a les significations indiquées ci-dessus,
avec 1 mol d'un composé phénolique de formule
R²-OX (III)
dans laquelle
R² a les significations indiquées ci-dessus et
X représente l'hydrogène ou un groupe -CO-O-alkyle en C₁-C₆, dont le groupe alkyle est à chaîne droite ou ramifiée,
de préférence avec une mole d'un composé phénolique de formule
R¹²-OH (V),
dans laquelle
R¹² représente un groupe phényle ou un groupe phényle portant un substituant alkyle en C₁-C₄, alcoxy en C₁-C₄ ou chloro,
en présence d'un catalyseur de transestérification connu en soi, à des températures de 80 à 350°C et des pressions allant de 10 mbar à 20 bar, caractérisé en ce que la réaction est réalisée dans un réacteur à colonne à bulles ou dans une série d'au moins deux colonnes à bulles par introduction du composé phénolique de formule (III) à l'état liquide dans la première colonne à bulles et du carbonate organique de formule (II), à l'état liquide ou gazeux, simultanément, dans chacune des colonnes à bulles mais de préférence uniquement dans la dernière colonne à bulles, le carbonate (II) étant vaporisé dans la colonne à bulles lorsqu'il a été introduit à l'état liquide, et évacuation de la dernière colonne à bulles des produits de réaction de formule (I) à l'état liquide et, simultanément, en tête de chacune des colonnes à bulles individuelles mais de préférence en tête de la première colonne à bulles, évacuation des produits de formule
R³-OX (IV)
dans laquelle R³ et X ont les significations indiquées ci-dessus,
à l'état gazeux.

2. Procédé selon la revendication 1, caractérisé en ce que la réaction est réalisée dans 1 à 18, et de préférence dans 2 à 12 colonnes à bulles.

3. Procédé selon la revendication 1, caractérisé en ce que la réaction est réalisée dans au moins deux réacteurs à colonne à bulles placés en série par introduction du carbonate organique de formule (II) dans la première colonne à bulles et évacuation de la dernière colonne à bulles du carbonate aromatique de formule (I) à l'état liquide et, en tête de la première colonne à bulles, du produit de formule (IV).

4. Procédé selon la revendication 1, caractérisé en ce que l'on opère à des températures de 100 à 250°C, de préférence de 120 à 240°C, et dans le cas d'une série de colonne à bulles, les températures dans ces colonnes sont identiques ou différentes.

5. Procédé selon la revendication 1, caractérisé en ce que l'on opère à des pressions de 0,05 à 15 bar, de préférence de 0,08 à 13 bar, et dans le cas d'une série d'une colonne à bulles, les pressions dans les diverses colonnes sont identiques ou différentes.

6. Procédé selon la revendication 1, caractérisé en ce que, dans le cas d'une série de colonne à bulles, la pression et la température s'abaissent de la première à la dernière colonne à bulles.

7. Procédé selon la revendication 1, caractérisé en ce que l'on utilise des colonnes à bulles à corps de garnissage, garnissages ordonnés ou plateaux perforés.

8. Procédé selon la revendication 1, caractérisé en ce que l'on combine une colonne à bulles ou une série de colonnes à bulles avec un ou plusieurs récipients à passage disposés à la suite.

9. Procédé selon la revendication 1, caractérisé en ce que l'on met en oeuvre le carbonate organique (II) en mélange avec 0 à 5 % en poids, de préférence 0,1 à 3 % en poids et plus spécialement 0,15 à 2 % en poids, par rapport au poids de (II), de l'alcool correspondant R³-OH.

10. Procédé selon la revendication 1, caractérisé en ce que, en plus des composants de départ, et à un endroit quelconque de la colonne à bulles ou de la série de colonnes à bulles, on introduit avec le carbonate de formule (II) ou séparément de ce carbonate un solvant inerte qui vaporise dans le mélange de réaction ou un gaz inerte, formant de préférence un azéotrope avec le produit de formule (IV).
